# DEMANDE DE BREVET EUROPEEN

(11) **EP 1 415 991 A1**
(43) Date de publication de la demande: **06.05.2004**
(21) Numéro de dépôt: 03292654.5
(22) Date de dépôt: 23.10.2003
(51) Int. Cl.: C07D 263/44, C07D 269/02

(54) **Procédé de purification des N-carboxyanhydrides**

(30) Priorité: 29.10.2002 FR 0213498
(71) Demandeur: ISOCHEM, 75004 Paris (FR)
(72) Inventeur: Paris, Antoine, 76000 Rouen (FR); Lebon, Marc, 91150 Ormoy la Riviere (FR)

(57) **Abrégé**

L'invention concerne un procédé de purification des N-carboxyanhydrides d'amino-acides qui est caractérisé en ce que l'on met les N-carboxyanhydrides en solution ou en suspension dans un milieu solvant non polaire, en contact avec de la silice.

On peut de plus améliorer encore la purification en traitant les N-carboxyanhydrides par une solution aqueuse d'un acide minéral ou organique.

Les N-carboxyanhydrides ainsi traités ne sont plus colorés.

## Description

L'invention concerne un procédé de purification des N-carboxyanhydrides d'amino-acides.

Les N-carboxyanhydrides (abréviation NCA) dérivés d'aminoacides notamment des α-, β-, ou γ- amino-acides sont des intermédiaires très utiles en raison de l'activation de la fonction acide et de la protection de la fonction amine qu'ils présentent. Ils permettent en effet la réaction de cette fonction acide avec toute entité nucléophile. Ainsi l'obtention de la fonction amide par réaction avec une fonction amine est facilitée. De ce fait, ils polymérisent facilement et sont utilisés pour former des peptides. La liaison ester par réaction avec un alcool se crée également aisément. Ils sont aussi intéressants lorsqu'on souhaite réduire une fonction acide. Leurs caractéristiques sont plus particulièrement décrites dans l'ouvrage de référence : ''α-Aminoacid-N-Carboxyanhydrides and Related Heterocycles'' par H.R. Kricheldorf-1987 - Springer-Verlag.

Pour la plupart de leurs applications, il est nécessaire qu'ils soient très purs, en particulier non colorés, exempts du stéréoisomère non souhaité et débarrassés des composés de départ n'ayant pas réagi ainsi que des sous-produits de la réaction.

Les procédés classiques de purification tels que la recristallisation ou la décoloration en présence de charbon actif ne donnent pas satisfaction. La technique de passage des composés au travers d'une colonne remplie est une technique de laboratoire qui ne peut pas être utilisée industriellement. Elle nécessite de très grandes quantités du produit de remplissage et de grandes quantités d'éluants particuliers qu'il faut choisir en fonction des vitesses d'élution des impuretés et du produit à récupérer ainsi qu'une surveillance étroite. Ces grandes quantités d'éluants et de produits de remplissage doivent être ensuite éliminées.

Les N-carboxyanhydrides qui polymérisent facilement sont également très sensibles à l'hydrolyse. En raison de ces caractéristiques, un traitement ultérieur de purification après leur obtention pose problème.

Il existait par conséquent un besoin de trouver un procédé de traitement des N-carboxyanhydrides utilisable à l'échelon industriel et qui permette de les obtenir sans coloration, plus purs et avec un très bon rendement.

Le procédé de purification des N-carboxyanhydrides selon la présente invention est caractérisé en ce que l'on met le ou les N-carboxyanhydrides d'amino-acides à purifier, en solution ou en suspension dans un milieu solvant non polaire, en contact avec de la silice ajoutée dans le milieu ou constituant un lit fixe.

A l'issue de ce traitement la coloration des N-carboxyanhydrides a pratiquement disparu. Ils n'ont pas été dégradés. Leur pureté est améliorée. Les composés optiquement actifs n'ont pas subi de racémisation. Des polymères ne se sont pas formés. Ce dernier résultat est surprenant car la silice est habituellement connue pour favoriser la polymérisation des N-carboxyanhydrides. Le rendement est excellent, souvent supérieur à 99%.

On a de plus trouvé que la purification peut encore être améliorée en soumettant les N-carboxyanhydrides, purifiés comme précédemment indiqué au moyen de la silice, à un traitement par un acide minéral ou organique.

Ce procédé permet d'éliminer les traces de matières organiques ou minérales restant encore dans les N-carboxyanhydrides.

Les N-carboxyanhydrides qui sont purifiés par le procédé de la présente invention sont tous les N-carboxyanhydrides d'amino-acides naturels ou synthétiques. Ils sont habituellement représentés par la formule 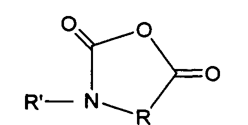 dans laquelle R représente le radical central compris entre la fonction acide et la fonction amine de l'amino-acide, éventuellement modifié, R' représente un atome hydrogène ou le radical porté au départ par l'atome d'azote de l'amino-acide ou qui a été fixé ou modifié ultérieurement et qui peut être lié à R, notamment lorsque les amino-acides sont cycliques.

En particulier, ce sont les N-carboxyanhydrides des α-, β-, ou γ- amino-acides et plus particulièrement des α-amino-acides.

Les groupes réactifs présents dans les composés sont sous forme protégée ou non, comme il est habituel.

Les N-carboxyanhydrides peuvent être sous leurs différentes formes et notamment lorsqu'ils possèdent un ou plusieurs carbones asymétriques, sous leurs différentes formes stéréochimiques telles que racémiques, énantiomères ou diastéréoisomères. Le procédé de purification selon l'invention n'entraîne pas de racémisation et les stéréoisomères de même configuration que celle de départ pourront être obtenus.

Le procédé convient particulièrement bien pour la purification de composés tels que ceux décrits dans la demande de brevet FR n° 2 815 962 ou le brevet US 6 479 665.

Selon le procédé de l'invention, les N-carboxyanhydrides à purifier doivent être mis en contact avec de la silice.

Pour favoriser le contact entre les N-carboxyanhydrides et la silice, la purification a lieu au moyen d'un milieu solvant des N-carboxyanhydrides et non polaire. Comme solvants non polaires les hydrocarbures aromatiques conviennent bien, en particulier le toluène ou le xylène.

Les N-carboxyanhydrides peuvent être complètement dissous dans le milieu solvant ou former une suspension dans celui-ci. Généralement, on préfère qu'ils soient en grande partie dissous et le choix du solvant sera fonction du ou des N-carboxyanhydrides à purifier.

On a aussi trouvé que la mise en contact devait être faite par ajout de la silice dans le milieu contenant les N-carboxyanhydrides à purifier ou en mettant la silice sous forme de lit fixe.

Selon le procédé de l'invention, des quantités de silice très faibles peuvent être utilisées telles que de 0,5 à 10 % en poids (bornes comprises) par rapport au poids de N-carboxyanhydride(s) à purifier.

Lorsque la silice constitue un lit fixe, il peut être placé à la sortie du réacteur et le mélange formé par le ou les N-carboxyanhydrides passe alors dans le lit fixe.

Selon la variante préférée du procédé, le mélange du ou des N-carboxyanhydrides, de la silice et du milieu solvant peut être simplement réalisé en le plaçant par exemple sous agitation.

Comme silice, on utilise de préférence les silices appartenant à la catégorie connue sous la dénomination gel de silice, par exemple telles que celles utilisées classiquement pour la chromatographie.

Comme il est connu, les N-carboxyanhydrides sont très sensibles à l'hydrolyse. Pour cette raison le traitement est de préférence effectué sous atmosphère anhydre, par exemple sous atmosphère d'azote.

La durée du contact, les quantités de silice et de solvant utilisés dépendent des N-carboxyanhydrides, de l'importance de la coloration à faire disparaître et de la méthode du contact utilisée.

Si nécessaire, le mélange peut être chauffé à une température qui ne dégrade pas les N-carboxyanhydrides.

Des quantités de silice inférieures à 5%, la température ambiante, un temps de contact d'une à quelques heures sont généralement suffisants lorsqu'on choisit d'ajouter la silice dans le mélange.

Le traitement terminé, on récupère les N-carboxyanhydrides selon les méthodes classiques. Notamment, après avoir séparé la silice du mélange, on fait précipiter les N-carboxyanhydrides en éliminant le solvant ou en ajoutant un liquide non-solvant des N-carboxyanhydrides, après avoir éventuellement enlevé une partie du solvant, par exemple par distillation. Comme liquide de précipitation, on peut citer les alcanes tels que l'heptane, le cyclohexane, les éthers tels que l'éther de méthyle et de tertio-butyle, l'éther de diisopropyle.

On constate que les N-carboxyanhydrides récupérés ne sont plus colorés et sont généralement des solides de couleur blanche. Les analyses montrent que la pureté des composés augmente et qu'il n'y a pas eu de racémisation des isomères optiques. Les rendements sont bons, souvent de l'ordre de 99%.

Pour améliorer encore ces résultats, si nécessaire, on peut après avoir traité les N-carboxyanhydrides avec la silice, les mettre en contact une ou plusieurs fois avec une solution aqueuse d'un acide minéral ou organique.

De préférence, on effectue ce traitement à un pH de 1 à 2.

Comme acides, on peut citer les acides minéraux tels que l'acide chlorhydrique, l'acide sulfurique et les acides organiques tels que l'acide acétique, l'acide citrique.

La concentration de l'acide dans la solution aqueuse est de préférence comprise entre 0,5% p/p et 5% p/p, plus particulièrement entre 0,5% p/p et 1,5% p/p.

De préférence, on utilise un acide minéral et plus particulièrement l'acide chlorhydrique.

Il est préférable d'effectuer le traitement à une température basse comprise entre 0°C et 15°C et de préférence entre 0°C et 5°C.

La solution d'acide est généralement mélangée avec la solution ou la suspension des N-carboxyanhydrides après en avoir extrait la silice. On peut éventuellement avant le traitement changer de milieu solvant. Le traitement terminé, la phase aqueuse est éliminée par exemple par décantation ou par distillation azéotropique avec le solvant.

Les N-carboxyanhydrides purifiés sont récupérés comme précédemment indiqué de façon connue. Ce traitement supplémentaire permet d'améliorer la pureté chimique des N-carboxyanhydrides par élimination plus poussée de traces de contaminants.

Ce résultat est surprenant car on pouvait craindre que les N-carboxyanhydrides soient hydrolysés et qu'en conséquence, ils contiennent beaucoup plus d'impuretés.

Les exemples qui suivent illustrent l'invention sans toutefois la limiter.

### Exemple 1 :

Dans un réacteur de 1 litre équipé, agité et sous courant d'azote, on introduit 600 ml de toluène et 150 g (0,491 mol) de N-[1-(S)-éthoxycarbonyl-3-phénylpropyl]-L-alanyl-N-carboxyanhydride (abréviation : EPAL-NCA), de couleur grisâtre, légèrement rosée. On introduit ensuite 1,5 g de gel de silice (70-200 µm) de la marque Millipore et on agite le mélange pendant environ 1 heure.

On filtre le mélange et on rince la silice au toluène. Puis on concentre le filtrat jaune pâle recueilli par distillation sous pression réduite.

On ajoute ensuite dans le milieu de l'heptane B. Un précipité se forme. On refroidit le milieu pour faire complètement précipiter l'EPAL-NCA.

On filtre la suspension et on rince le solide avec de l'heptane B. On sèche ensuite en étuve sous vide le gâteau blanc humide.

On recueille alors 148,7 g (rendement 99,1%) d'EPAL-NCA purifié, solide blanc.

Les caractéristiques de l'EPAL-NCA de départ et obtenu après purification sont rassemblées dans le tableau suivant :

| Caractéristiques | EPAL-NCA de départ | EPAL-NCA purifié |
|---|---|---|
| Apparence | Solide gris légèrement rosé | Solide blanc |
| Pouvoir rotatoire α_{D} ²⁵ (c=2, CH₃CN) | +12,3° | +12,4° |
| Pureté par HPLC | 99,80% | 99,90% |

### Exemple 2 :

On traite comme à l'exemple 1, 200 g d'EPAL-NCA dans 800 ml de toluène avec 2,0 g de gel de silice (40-70 µm).

On filtre le mélange pour séparer la silice et on la rince au toluène.

On mélange, sous agitation, 2 fois le filtrat avec 200 ml d'une solution aqueuse à 1 % (p/p) d'acide chlorhydrique, dont le pH est d'environ 1,5, à une température de 0°-5°C et on enlève à chaque fois la phase aqueuse.

On récupère ensuite l'EPAL-NCA en opérant comme à l'exemple 1, après concentration du milieu et au moyen d'heptane B.

On obtient 185,6 g d'EPAL-NCA sec (rendement 92,8%) solide de couleur blanche. Ses caractéristiques de départ et après purification sont rassemblées dans le tableau suivant :

| Caractéristiques | EPAL-NCA De départ | EPAL-NCA purifié |
|---|---|---|
| Apparence | Solide gris | Solide blanc |
| Pouvoir rotatoire α _{D} ²⁵ (c=2, CH₃CN) | +12,2° | +12,4° |
| Pureté par HPLC | 99,85 % | 99,83 % |

### Exemple 3 :

On traite, comme à l'exemple 2, 200 g d'EPAL-NCA, contenant 0,5% d'EPAL, dans 800 ml de toluène avec 2,0 g de gel de silice (40-70 µm).

On filtre le mélange pour séparer la silice et on la rince au toluène.

On lave le filtrat 2 fois avec une solution à 1% (p/p) d'acide chlorhydrique comme à l'exemple 2.

On récupère ensuite l'EPAL-NCA en opérant comme à l'exemple 1, après concentration du milieu et au moyen d'heptane B.

On obtient 184 g d'EPAL-NCA sec (rendement 92%) solide de couleur blanche. Ses caractéristiques de départ et après purification sont rassemblées dans le tableau suivant :

| Caractéristiques | EPAL-NCA de départ | EPAL-NCA purifié |
|---|---|---|
| Apparence | Solide gris | Solide blanc |
| Pureté par HPLC | 99,5 % | 99,9 % et absence d'EPAL |

## Revendications

1. Procédé de purification des N-carboxyanhydrides d'amino-acides, **caractérisé en ce que** l'on met le ou les N-carboxyanhydrides d'amino-acides, en solution ou en suspension dans un milieu solvant non polaire, en contact avec de la silice ajoutée dans le milieu ou constituant un lit fixe.

2. Procédé selon la revendication 1, **caractérisé en ce que** la quantité de silice utilisée est choisie dans la gamme allant de 0,5 à 10 % en poids par rapport au poids de N-carboxyanhydrides à purifier.

3. Procédé selon la revendication 1 ou 2, **caractérisé en ce que** le contact est réalisé par le mélange dans le milieu solvant non polaire de la silice avec le ou les N-carboxyanhydrides en solution ou en suspension.

4. Procédé selon l'une quelconque des revendications précédentes, **caractérisé en ce que** la silice utilisée est choisie dans la catégorie appelée gel de silice.

5. Procédé selon l'une quelconque des revendications précédentes, **caractérisé en ce que** le milieu solvant est choisi parmi les hydrocarbures aromatiques.

6. Procédé selon l'une quelconque des revendications précédentes, **caractérisé en ce qu'**on traite le ou les N-carboxyanhydrides purifiés avec une solution aqueuse d'un acide minéral ou organique.

7. Procédé selon la revendication 6, **caractérisé en ce que** le pH du milieu pendant le traitement est compris entre 1 et 2.

8. Procédé selon la revendication 6 ou 7, **caractérisé en ce que** le traitement est effectué à une température comprise entre 0°C et 15°C.

9. Procédé selon l'une quelconque des revendications 6 à 8, **caractérisé en ce que** l'acide utilisé est un acide minéral.

10. Procédé selon la revendication précédente, **caractérisé en ce que** l'acide minéral est l'acide chlorhydrique.
